# EUROPÄISCHE PATENTANMELDUNG

(11) **EP 1 634 868 A2**
(43) Veröffentlichungstag der Anmeldung: **15.03.2006**
(21) Anmeldenummer: 05104858.5
(22) Anmeldetag: 03.06.2005
(51) Int. Cl.: C07C 265/14, C07C 263/04, C07C 269/04

(54) **Mehrstufiges Verfahren zur kontinuierlichen Herstellung von cycloaliphatischen Diisocyanaten**

(30) Priorität: 28.07.2004 DE 102004036499
(71) Anmelder: Degussa AG, 40474 Düsseldorf (DE)
(72) Erfinder: Kohlstruk, Stephan, 48249 Dülmen (DE); Kreczinski, Manfred, 44652 Herne (DE); Michalczak, Hans-Werner, 44651 Herne (DE)

(57) **Zusammenfassung**

Die Erfindung betrifft ein mehrstufiges Verfahren zur kontinuierlichen und phosgenfreien Herstellung von cycloaliphatischen Diisocyanaten.

## Beschreibung

Die Erfindung betrifft ein mehrstufiges Verfahren zur kontinuierlichen und phosgenfreien Herstellung von cycloaliphatischen Diisocyanaten.

Der synthetische Zugang zu Isocyanaten kann über eine Reihe unterschiedlicher Routen erfolgen. Älteste und auch heute noch vorherrschende Variante zur großtechnischen Herstellung von Isocyanaten ist die so genannte Phosgenroute. Grundlage dieses Verfahrens ist Umsetzung von Aminen mit Phosgen. Nachteil des Phosgenverfahrens ist der Einsatz von Phosgen, dass aufgrund seiner Toxizität und Korrosivität besonders hohe Anforderungen an seine Handhabung im industriellen Maßstab stellt.

Es gibt mehrere Verfahren, die Verwendung von Phosgen zur Herstellung von Isocyanaten in technischen Größenordnungen zu umgehen. Der Begriff phosgenfreies Verfahren wird häufig im Zusammenhang mit der Überführung von Aminen in Isocyanate unter Einsatz alternativer Carbonylierungsmittel, z. B. Harnstoff oder Dialkylcarbonat, benutzt (EP 018 586, EP 355 443, US 4,268,683, EP 990 644).

Grundlage der so genannten Harnstoffroute ist die Harnstoff-vermittelte Überführung von Diaminen in Diisocyanate über einen zweistufigen Prozess. Im ersten Schritt wird ein Diamin mit Alkohol in Gegenwart von Harnstoff oder Hamstoff-Äquivalenten (z. B. Alkylcarbonate, Alkylcarbamate) zu einem Diurethan umgesetzt, welches üblicherweise eine Zwischenreinigungstufe durchläuft und dann im zweiten Schritt thermisch in Diisocyanat und Alkohol gespalten wird (EP 355 443, US 4,713,476, US 5,386,053). Alternativ kann der eigentlichen Urethanbildung auch die separate Herstellung eines Diharnstoffs durch gezielte Umsetzung des Diamins mit Harnstoff vorgeschaltet sein (EP 568 782). Denkbar ist auch eine zweistufige Sequenz aus partieller Umsetzung von Harnstoff mit Alkohol im ersten und anschließender Zudosierung und Urethansierung des Diamins im zweiten Schritt (EP 657 420).

Die thermische Spaltung von Urethanen in die entsprechenden Isocyanate und Alkohole ist seit langem bekannt und kann sowohl in der Gasphase bei hohen Temperaturen als auch bei relativ niedrigen Temperaturen in der Flüssigphase durchgeführt werden. Problematisch ist jedoch bei beiden Verfahrensweisen, dass durch die thermische Belastung grundsätzlich auch unerwünschte Nebenreaktionen stattfinden, die zum einen die Ausbeute mindern und zum anderen zur Bildung verharzender Nebenprodukte führen, die den Ablauf eines technischen Prozesses durch Belegungen und Verstopfungen in Reaktoren und Aufarbeitungsvorrichtungen erheblich stören.

Es hat daher nicht an Vorschlägen gefehlt, durch chemische und verfahrenstechnische Maßnahmen Ausbeuteverbesserungen zu erzielen und die unerwünschte Nebenproduktbildung einzuschränken. So wird in einer Reihe von Dokumenten der Einsatz von Katalysatoren beschrieben, die die Spaltreaktion der Urethane beschleunigen (DE 10 22 222, US 3,919,279, DE 26 35 490). Tatsächlich gelingt es in Gegenwart geeigneter Katalysatoren - hierbei handelt es sich um eine Vielzahl basischer, saurer sowie metallorgansicher Verbindungen - durchaus, die Isocyanatausbeute im Vergleich zur unkatalysierten Variante zu steigern. Die Bildung unerwünschter Nebenprodukte kann jedoch auch durch die Anwesenheit eines Katalysators nicht vermieden werden. Dasselbe gilt für die zusätzliche Verwendung von inerten Lösemitteln, wie sie ebenfalls in der US 3,919,279 und DE 26 35 490 empfohlen werden, um eine gleichmäßige Verteilung der zugeführten Wärme und des Katalysators im Reaktionsmedium zu gewährleisten. Grundsätzlich hat die Verwendung von unter Rückfluss siedenden Lösemitteln jedoch eine Reduzierung der Raum/Zeit-Ausbeute an Isocyanaten zur Folge und ist darüber hinaus mit dem Nachteil eines zusätzlichen hohen Energieaufwands behaftet.

In der EP 054 817 angeführte Beispiele zur thermisch geführten katalysierten Spaltung von Monourethanen beschreiben die Teilausschleusung des Reaktionsgemisches zur Abtrennung der im Zuge der Urethanspaltung entstehenden verharzenden Nebenprodukte. Diese Prozedur dient der Vermeidung von Belegungen und Verstopfungen in Reaktoren und Aufarbeitungseinrichtungen. Hinweise, die auf eine Ausbeute steigernde Verwertung der Teilausschleusung hindeuten, gibt es nicht. Die EP 61 013 beschreibt einen ähnlichen Lösungsansatz, wobei die Thermolyse in diesem Fall in Gegenwart von Lösemitteln durchgeführt wird, deren Aufgabe offenbar in einer besseren Aufnahme der schwerflüchtigen Nebenprodukten besteht. Auch hier wird die Teilausschleusung nicht im Sinne einer Ausbeuteoptimierung verwertet.

Aus der EP 355 443 ist nun bekannt, dass eine Ausbeutesteigerung erzielt werden kann, wenn die während der Spaltung von Diurethanen im Spaltreaktor entstehenden höhermolekularen, verwertbaren und nicht verwertbaren Nebenprodukte zur Gewährleistung einer störungsfreien und selektiven Reaktion möglichst kontinuierlich aus dem Reaktor ausgeschleust werden und anschließend in Gegenwart von Alkohol zum großen Teil umgesetzt und dann in die Diurethanherstellung zurückgeführt werden. Die beschriebene Verfahrensweise ist mit einem hohen Energieaufwand verbunden, da die Abtrennung nicht verwertbarer Nebenprodukte aus dem Austrag der Diurethanherstellung destillativ erfolgt, wobei das gesamte Diurethan verdampft werden muss. Im Unterschied zur EP 355 443 wird der Urethanisierungsaustrag beim Verfahren der EP 566 925 in zwei Teilströme aufgeteilt, von denen nur einer destillativ von seinen hochsiedenden, nicht verwertbaren Nebenprodukten befreit wird, bevor die vereinigten Diurethanströme der Deblockierungsreaktion im Spaltreaktor zugeführt werden. Zudem wird die kontinuierliche Spaltreaktorausschleusung bei der EP 566 925 direkt, d. h. ohne Reurethanisierungsschritt, in die Diurethansynthese zurückgeführt.

Die Herstellung der Diurethane in einer Eintopfreaktion aus Harnstoff, Diamin und Alkohol unter gleichzeitiger Abtrennung von Ammoniak ist gängige Praxis und wird in einer Reihe von Patenschriften beschrieben (EP 018 568, EP 355 443, EP 566 925). Nachteilig ist, dass durch die simultane Umsetzung von Harnstoff, Alkohol und Diamin zwangsläufig und in größerer Menge Nebenprodukte gebildet werden, die die Selektivität der Umsetzung beeinträchtigen und die vor der thermischen Deblockierung der Diurethane abgetrennt werden müssen. Die EP 568 782 beansprucht daher ein kontinuierliches Verfahren zur Herstellung von (cyclo)aliphatischen Diisocyanaten, welches im wesentlichen drei Hauptschritte umfasst, von denen der erste die Bildung von Bisharnstoffen, der zweite die Bildung von Diurethanen aus den Bisharnstoffen und der dritte die Spaltung der Diurethane in flüssiger Phase zu den gewünschten Diisocyanaten beschreibt - d. h., die Herstellung des Diurethans erfolgt in zwei getrennten Stufen. Nach der Lehre der EP 568 782 wird der Austrag der Reaktionssequenz aus Bisharnstoff-Bildung und anschließender Diurethansynthese zunächst destillativ von Leicht- und Mittelsiedern wie Alkoholen, Carbamaten und Carbonaten befreit und der Hochsieder im Diurethan danach durch Kurzwegverdampfung abgetrennt. Das Diurethan wird thermisch deblockiert und ein Teil des Spaltsumpfes wird kontinuierlich ausgeschleust, mit Alkohol reurethanisiert und wieder in die Diurethansynthesestufe zurückgeführt.

Überraschenderweise wurde gefunden, daß es bei Einsatz von cycloaliphatischen Diaminen vorteilhaft ist, die cycloaliphatischen Diurethane durch zweistufige und somit über Bisharnstoff verlaufende Umsetzung von cycloaliphatischen Diaminen mit Alkohol und Harnstoff herzustellen, sie von Leicht- und Mittelsiedern zu befreien, die so gereinigten cycloaliphatischen Diurethane unter Freisetzung des gewünschten cycloaliphatischen Diisocyanats thermisch zu spalten, einen Teil des Spaltsumpfes aus der Spaltapparatur kontinuierlich auszuschleusen und mit Alkohol zu reurethanisieren, hieraus Hochsiederkomponenten abzutrennen und das so gereinigte Reurethanisat und in den Prozess zu recyclieren. Es hat sich herausgestellt, dass auf diese Weise zum einen eine vergleichsweise niedrige stationäre Konzentration an Hochsiederkomponenten über die gesamte Sequenz Diurethan-Synthese, Diurethan-Reinigung und Diurethan-Spaltung realisiert wird, so dass Ablagerungen, die insbesondere durch die von Natur aus hochviskosen Hochsiederkomponenten begünstigt werden, weitgehend vermieden werden können und auch langfristig eine gute Anlagenverfügbarkeit und eine gute Verfahrensausbeute gewährleistet sind. Zum anderen hat die der thermischen Spaltreaktion nachgeschaltete Sequenz aus Reurethanisierung und Hochsiederabtrennung den Vorteil, dass im Vergleich zur üblichen Vorgehensweise, bei der der Hochsieder vor der Diurethanspaltung abgetrennt wird, die in die Dampfphase zu überführende Diurethanmenge signifikant verringert ist, wodurch Investment- und Energiekosten eingespart werden können.

Gegenstand der Erfindung ist ein mehrstufiges Verfahren zur kontinuierlichen Herstellung von cycloaliphatischen Diisocyanaten, durch Umsetzung von cycloalipahtischen Diaminen mit Kohlensäurederivaten und Alkoholen zu cycloaliphatischen Diurethanen und anschließenden thermischen Spaltung der Diurethane zu cycloaliphatischen Diisocyanten, dadurch gekennzeichnet, dass die Bildung der Diurethane zweistufig ausgeführt ist, das von Leicht-, Mittelsiedern befreite Diurethan unter Freisetzung des gewünschten Diioscyanats thermisch gespalten wird, ein Teil des Spaltsumpfes der Spaltapparatur kontiniuerlich ausgeschleust und mit Alkohol reurethanisiert wird, hieraus die Hochsiederkomponenten abgetrennt werden und das so gereinigte Reurethanisat in den Prozess recycliert wird.

Gegenstand der Erfindung ist auch ein mehrstufiges Verfahren zur kontinuierlichen Herstellung von cycloaliphatischen Diisocyanaten der Formel (I)

OCN-R-NCO

wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, durch Umsetzung cycloaliphatischer Diamine mit Kohlensäurederivaten und Alkoholen zu Diurethanen, und deren thermische Spaltung,
wobei
a) cycloaliphatische Diamine der Formel (II)

   H₂N-R-NH₂

   wobei R für einen zweiwertigen cycloaliphatischen Kohlenwasserstoffrest mit 4 bis 18, vorzugsweise 5 bis 15 Kohlenstoffatomen steht, wobei die beiden Stickstoffatome direkt an mindestens einem Kohlenwasserstoffcyclus gebunden und zwischen ihnen mindestens 3 Kohlenstoffatome angeordnet sind, mit Harnstoff und in Gegenwart von Alkoholen der Formel (III)

   R¹-OH

   wobei R' für einen Rest steht, wie er nach Entfernung der Hydroxylgruppe aus einem primären oder sekundären (cyclo)aliphatischen Alkohol mit 3 bis 8 Kohlenstoffatomen verbleibt,
   in Abwesenheit oder Gegenwart von Katalysatoren zu Cycloalkylenbisharnstoffen der Formel (IV)

   H₂N-OC-HN-R-NH-CO-NH₂

   wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden R flankierenden Stickstoffatome direkt an einem Kohlenwasserstoffcyclus gebunden und zwischen ihnen mindestens 3 Kohlenstoffatome angeordnet sind, umgesetzt werden und das entstehende Ammoniak gleichzeitig kontinuierlich abgetrennt wird;
b) der anfallende Roh-Cycloalkylenbisharnstoff in einem zweiten Reaktor mit dem in a) als Lösemittel eingesetzten Alkohol der Formel (III) unter kontinuierlicher Austreibung des freigesetzten Ammoniaks in Cycloalkylendiurethan der Formel (V)

   R¹O-OC-HN-R-NH-CO-OR¹

   überführt wird;
c) aus der erhaltenen Reaktionsmischung der Alkohol, die Dialkylcarbonate und/oder Carbamidsäurealkylester abgetrennt und der Alkohol in die Reaktionsstufe a) zurückführt wird;
d) auf eine Abtrennung der in der erhaltenen Reaktionsmischung gegebenenfalls enthaltenen hochsiedenden Rückstände vollständig oder partiell verzichtet wird;
e) die über die Schritte c) und d) aufgereinigte Diurethane enthaltende Reaktionsmischung in Gegenwart eines Katalysators kontinuierlich und lösemittelfrei bei Temperaturen von 180 bis 280 °C, vorzugsweise 200 bis 260 °C, und unter einem Druck von 0,1 bis 200 mbar, vorzugsweise 0,2 bis 100 mbar thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig aus dem Sumpf ausgeschleust wird;
f) die Spaltprodukte durch Rektifikation in ein rohes Diisocyanat und Alkohol getrennt werden;
g) das rohe cycloaliphatische Diisocyanat durch Destillation gereinigt und die Reinproduktfraktion isoliert wird;
h) die Sumpfausschleusung aus e) partiell oder vollständig mit dem Alkohol aus f) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt wird und das Molverhältnis von NCO-Gruppen und OH-Gruppen bis zu 1 : 100, vorzugsweise 1 : 20 und besonders bevorzugt 1 : 10 beträgt;
i) der Wertstoffstrom aus h) in einen Wertstoff- und einen Abfallstrom aufgetrennt und der an Hochsiederkomponenten reiche Abfallstrom aus dem Prozess ausgeschleust und verworfen wird;
j) ein Teil der Sumpffraktion der Reindestillation g) kontinuierlich ausgeschleust und in die Spaltreaktion e) und/oder in die Urethanisierungsstufe h) geführt wird;
k) optional die bei der Reindestillation des rohen cycloaliphatischen Diisocyanats anfallende Kopffraktion ebenfalls in die Urethanisierungsstufe h) zurückgeführt wird;
l) der aufgereinigte Reurethanisatstrom aus i) in Stufe b) und/oder c) oder e) zurückgeführt wird.

Nach dem erfindungsgemäßen Verfahren können cycloaliphatische Diisocyanate im kontinuierlichen Betrieb problemlos mit sehr guter Ausbeute hergestellt werden. Vorteilhaft bei dem erfindungsgemäßen Mehrstufenverfahren ist insbesondere die Tatsache, daß auch bei Einsatz von cycloaliphatischen Diaminen der Formel (II) als Ausgangsmaterial für die kontinuierliche Diisocyanatsynthese Ablagerungen, die insbesondere durch die von Natur aus hochviskosen Hochsiederkomponenten begünstigt werden, weitgehend vermieden werden können und auch langfristig eine gute Anlagenverfügbarkeit und eine gute Verfahrensausbeute gewährleistet sind. Weiterhin ist es ein Vorteil des erfindungsgemäßen Mehrstufenverfahrens, dass es erlaubt, die Menge des in die Dampfphase zu überführenden Diurethans auf ein Minimum zu verringern und auf diese Weise den notwendigen Energieaufwand beschränkt.
a) Zur Herstellung der Cycloalkylenbisharnstoffe der Formel (IV) in der Reaktionsstufe a) werden die cycloaliphatischen Diamine der Formel (II), mit Harnstoff in Gegenwart eines Alkohols der Formel (III), gegebenenfalls auch Mischungen solcher Alkohole, in einem Reaktor bei 100 bis 145 °C und einem Druck von 0,7 bis 1,8 bar umgesetzt, wobei das gebildete Ammoniak kontinuierlich ausgetrieben wird. Die Umsetzung erfolgt bevorzugt in einem Destillationsreaktor, wobei die Edukte in einem Molverhältnis von Diamin : Harnstoff : Alkohol von 1 : 2,0 bis 2,4 : 3 bis 10 kontinuierlich auf den obersten Boden aufgegeben werden und das freigesetzte Ammoniak durch Alkoholbrüden, die im Sumpf des Destillationsreaktor eingeführt werden, ausgetrieben wird. Die erforderliche Verweilzeit beträgt 4 bis 10 Stunden, vorzugsweise 5 bis 9 Stunden. Die zum Austreiben des Ammoniaks im Sumpf eingebrachte Alkoholmenge beträgt 0,05 bis 3 kg/kg, vorzugsweise 0,1 bis 1 kg/kg Bisharnstoff, wobei die so eingetragene Alkoholmenge zusammen mit dem gebildeten Ammoniak am Kopf abgezogen, nach partieller Kondensation in einer Alkoholrückgewinnungskolonne vom restlichen Ammoniak befreit und in den Sumpf zurückgeführt wird.
b) Der im Sumpf des Destillationsreaktors anfallende, in Alkohol gelöste rohe Cycloalkylenbisharnstoff wird kontinuierlich in einen zweiten Reaktor gefahren, in dem die Umsetzung zum Diurethan bei erhöhter Temperatur und erhöhtem Druck erfolgt, wobei wiederum Ammoniak freigesetzt wird, welches aus Gründen des chemischen Gleichgewichts aus dem Reaktionsgemisch entfernt werden muss. Die weitere Umsetzung des rohen Cycloalkylenharnstoffs aus a) erfolgt vorzugsweise in einem Druckdestillationsreaktor und bei einem molaren Verhältnis von Bisharnstoff zu Alkohol von 1 : 5 bis 12. Dabei wird der Stoffstrom aus a) vorzugsweise kontinuierlich auf den obersten Boden des Druckdestillationsreaktors gefahren. Die Umsetzung findet in Abwesenheit oder Gegenwart von Katalysatoren bei Reaktionstemperaturen von 140 bis 270 °C, vorzugsweise 160 bis 250 °C und unter einem Druck, der 5 bis 20 bar, vorzugsweise 7 bis 15 bar beträgt, innerhalb von 2 bis 20 Stunden, vorzugsweise 8 bis 15 Stunden, statt. Die kontinuierlich Austreibung des freigesetzten Ammoniaks wird unterstützt durch Alkoholbrüden, die im Sumpf des Druckdestillationsreaktors eingebracht und zweckmäßigerweise in einem am Sumpf der Kolonnne angebrachten Verdampfer erzeugt werden.
   Zur Erhöhung der Reaktionsgeschwindigkeit können die Diurethane in Gegenwart von Katalysatoren hergestellt werden. Als Katalysatoren eignen sich anorganische oder organische Verbindungen, die ein oder mehrere, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäß Handbook of Chemistry and Physics 14^{th} Edition, publiziert von Chemical Rubber Publishing Co. 2310 Superior Ave. N.E. Cleveland, Ohio, enthalten, beispielsweise Halogenide wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocaramate. Beispielhaft genannt seinen die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen, Kobalt und Nickel. Als typische Katalysatoren seinen beispielhaft folgende Verbindungen genannt: Lithiumethanolat, Lithiumbutanolat, Natriummethanolat, Kalium-tert.-butanolat, Magnesiumethanolat, Calciummethanolat, Zinn-(II)-chlorid, Zinn-(IV)-chlorid, Bleiacetat, Aluminiumtrichlorid, Bismuttrichlorid, Kupfer-(II)-acetat, Kupfer-(II)-chlorid, Zinkchlorid, Zinkoctoat, Titantetrabutanolat, Vanadiumtrichlorid, Vanadiumacetylacetoat, Mangan-(II)-acetat, Eisen-(II)-acetat, Eisen-(III)-acetat, Eisenoxalat, Cobaltchlorid, Cobaltnaphthenat, Nickelchlorid, Nickelnaphthenat sowie deren Mischungen. Die Katalysatoren können gegebenenfalls auch in Form ihrer Hydrate oder Ammoniakate zu Einsatz kommen.
   Ausgangsverbindungen für das erfindungsgemäße Verfahren sind Diamine der bereits obengenannten Formel (II), Alkohole der bereits obengenannten Formel (III) sowie Harnstoff. Geeignete Diamine der Formel (II) sind beispielsweise 1,4-Diaminocyclohexan, 4,4'-Methylendicyclohexyldiamin, 2,4-Methylendicyclohexyldiamin, 2,2'-Methylendicyclohexyldiamin und isomere cycloaliphatische Diamine sowie perhydriertes Methylendiphenyldiamin. Methylendiphenyldiamin (MDA) fällt herstellungsbedingt als Isomerenmischung aus 4,4'-, 2,4- und 2,2'-MDA an (s. z. B. DE 101 27 273). Perhydriertes Methylendiphenyldiamin wird durch vollständige Hydrierung von MDA erhalten und ist demzufolge eine Mischung aus isomeren Methylendicyclohexyldiaminen (H₁₂MDA), nämlich 4,4'-, 2,4- und 2,2'-H₁₂MDA und eventuell geringen Mengen an nicht vollständig umgesetzten (teil)aromatischen MDA. Bevorzugt werden als Diamine der Formel (II) 4,4'-Methylendicyclohexyldiamin, 2,4-Methylendicyclohexyldiamin und 2,2'-Methylendicyclohexyldiamin sowie auch beliebige Mischungen mindestens zweier dieser Isomere eingesetzt. Selbstverständlich können auch Diamine zum Einsatz gelangen, die von der Formel (II) abweichen. Beispielhaft seinen 1,3- und 1,4-Diaminomethylcyclohexan, Hexandiamin-1,6, 2,2,4- bzw. 2,4,4-Trimethylhexanamin-1,6 und 3-Aminomethyl-3,5,5-trimethylcyclohexylamin aufgeführt. Der Einsatz von Aminen, die von der Formel (II) abweichen, ist jedoch nicht bevorzugt.
   Als Alkohole der Formel (III) eignen sich beliebige aliphatische oder cycloaliphatische Alkohole, die unter Normaldruck einen unterhalb 190 °C liegenden Siedepunkt aufweisen. Beispielhaft genannt seinen C1-C6-Alkanole wie z. B. Methanol, Ethanol, 1-Propanol, 1-Butanol, 2-Butanol, 1-Hexanol oder Cyclohexanol. Bevorzugt wird 1-Butanol als Alkohol verwendet.
   Im Zuge der Umsetzung des Reaktionsgemisches wird Ammoniak freigesetzt, dessen Entfernung aus dem Reaktionsgleichgewicht sich als vorteilhaft erwiesen hat. Beim Austrag des Ammoniaks aus dem Reaktor ist darauf zu achten, dass die Wandtemperaturen des Reaktors und des Austragsrohres oberhalb von 60 °C liegen, damit eine Belegung durch Ammoniumcarbaminat, welches in minimalen Mengen aus Ammoniak und Kohlendioxid durch Zersetzung von Harnstoff gebildet wird, vermieden werden kann. Es hat sich beispielsweise bewährt, die Umsetzung in einem Druckdestillationsreaktor durchzuführen, wobei das Reaktionsgemisch im Gegenstrom zu im Sumpf eingebrachten Alkoholbrüden geführt wird und auf diese Weise eine derartig intensive Durchmischung der Flüssigkeit auf den Böden, die praktisch jeweils einer Kaskadenstufe entsprechen, erfolgt. Das am Kopf abgezogene, dampfförmige Gemisch aus Alkohol und Ammoniak kann, vorzugsweise unter dem Druck des Druckdestillationsreaktors und ohne es vorher zu kondensieren, in eine Destillationskolonne geführt werden, vom Ammoniak freien Alkohol zu gewinnen, der in den Sumpf des Druckdestillationsreaktors und der Kolonne zurückgeführt wird. Um eine Belegung des Rückflusskondensators mit Ammoniumcarbaminat zu verhindern, lässt man in diesem zur Einstellung der Temperatur am Kopf auf mindestens 60 °C einen entsprechenden Anteil an Alkohol zu.
c) Der überschüssige Alkohol, die Dialkylcarbonate, sofern solche gebildet wurden, oder Carbamidsäurealkylester oder Mischungen aus mindestens zwei dieser Komponenten werden einstufig, oder vorteilhafterweise zweistufig abgetrennt. Auf der ersten Stufe wird die Reaktionsmischung vom Druckniveau der Reaktionsstufe b) auf einen Druck von 1 bis 500 mbar, vorzugsweise 2 bis 150 mbar, entspannt und auf diese Weise in gasförmige Brüden, die die überwiegende Alkoholmenge sowie gegebenenfalls Dialkylcarbonate und/oder Carbamidsäurealkylester enthalten, und in einen flüssigen Austrag aufgetrennt. Im zweiten Schritt wird der flüssige Austrag durch Dünnschichtverdampfung bei 180 bis 250 °C, vorzugsweise 200 bis 230 °C, und einem Druck von 0,1 bis 20 mbar, vorzugsweise 1 bis 10 mbar, von gegebenenfalls vorhandenem restlichen Butanol sowie Mittelsiedern wie Dialkylcarbonaten und/oder Carbamidsäurealkylestern befreit, so dass der Rückstand im Wesentlichen aus dem monomeren Diurethan und gegebenenfalls hochsiedenden Oligomeren besteht. Die Brüden können nach weiterer destillativer Reinigung in die Reaktionsstufe a) zurückgeführt werden. Eine Rückführung der Dialkylcarbonate und/oder Carbamidsäure-alkylester in die Reaktionsstufe b) ist möglich aber nicht erforderlich.
d) Bevorzugt wird auf jedwede Abtrennung der in der Reaktionsmischung aus Stufe c) gegebenenfalls enthaltenen Hochsieder verzichtet. Sofern die unter i) beschriebene Auftrennung des Reurethanisatstroms aus Stufe h) jedoch nur mit einem Teilstrom, d. h. partiell, durchgeführt wird, kann es vorteilhaft sein, die anschließend erläuterten Wege zur Hochsiederabtrennung zu beschreiten:
   Optional kann der nach Abtrennung von Leicht- und Mittelsiedern erhaltene flüssige, die monomeren Diurethane, und gegebenenfalls hochsiedende Oligomere enthaltende Stoffstrom aus Schritt c), vorzugsweise mit Hilfe eines Dünnschicht- oder Kurzwegverdampfers, bei einer Temperatur von 180 bis 270 °C, vorzugsweise 200 bis 250 °C und unter einem Druck von 0,01 bis 10 mbar, vorzugsweise 0,02 bis 5 mbar destillativ in einen Wertstoffstrom, der die monomeren Diurethane und die leichter siedenden Nebenprodukte enthält, und einen nicht destillierbaren Nebenproduktstrom getrennt werden. Der die hochsiedenden Komponenten enthaltende, nicht destillierbare Nebenproduktstrom wird aus dem Herstellverfahren ausgeschleust und üblicherweise als stofflich nicht verwertbarer Rückstand verworfen.
   Optional kann der gegebenenfalls hochsiedende Oligomere enthaltende Stoffstrom aus Stufe c) vor seiner oben beschriebenen destillativen Aufreinigung auch in zwei Teilströme aufgeteilt werden, von denen einer direkt der Deblockierungsreaktion (siehe e)) zugeführt wird und der andere zunächst die soeben beschriebene Hochsiederabtrennung durchläuft.
e) Der die monomeren Diurethane und die leichter siedenden Nebenprodukte enthaltende Wertstoffstrom aus Stufe c) und optional aus Stufe d) wird in einer geeigneten Vorrichtung teilweise, lösemittelfrei in flüssiger Phase in Gegenwart von Katalysatoren bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch gespalten. Der Umsatz von Diurethan zu Diisocyanat in der Vorrichtung zur thermischen Spaltung kann in Abhängigkeit vom verwendeten Diurethan weitgehend frei gewählt werden und liegt üblicherweise in einem Bereich von 10 bis 95 Gew.-%, vorzugsweise 35 bis 85 Gew.-% der zugeführten Diurethanmenge (Feed). Der ungespaltende Anteil der Reaktionsmischung, der nicht umgesetzte Diurethane, hochsiedende Nebenprodukte und andere wieder verwertbare und unverwertbare Nebenprodukte enthält, wird kontinuierlich ausgeschleust. Die Menge der Ausschleusung richtet sich u.a. nach dem gewünschten Umsatz und der gewünschten Kapazität der Spaltreaktion und kann leicht experimentell ermittelt werden. Sie beträgt üblicherweise 10 bis 60 Gew.-%, vorzugsweise 15 bis 45 Gew.-%, bezogen auf den Feed.
   Als Katalysatoren zur chemischen Spaltung der Diurethane finden z. B. die vorgenannten, die Urethanbildung katalysierenden anorganischen und organischen Verbindungen Verwendung. Vorzugsweise werden Chloride des Zinks,Zinns oder Kupfers sowie Zink-, Mangan-, Eisen-, oder Cobaltoxide eingesetzt, wobei der Katalysator dem Massenstrom aus der Reinigungsstufe c) und optional d) vor dessen Zuführung in die Spaltung als 0,01 bis 25 Gew.-%ige, vorzugsweise 0,05 bis 10 Gew.-%ige Lösung oder Suspension in dem Alkohol, der auch zur Urethanherstellung verwendet wird, in einer Menge von 5 bis 400 ppm, vorzugsweise 10 bis 100 ppm, zudosiert wird.
   Als Spaltvorrichtungen eigenen sich beispielsweise zylinderförmige Spaltreaktoren, wie z. B. Röhrenöfen oder vorzugsweise Verdampfer, beispielsweise Fallfilm-, Dünnschicht- oder Bulkverdampfer, wie z. B. Robertverdampfer, Herbertverdampfer, caddle-typ-Verdampfer, Oskarverdampfer und Heizkerzenverdampfer.
   Prinzipiell geht es darum, die mittlere Verweilzeit der Isocyanatgruppen, die bei Deblockierung des Alkohols zwangsläufig freigesetzt werden, in der Spaltzone möglichst gering zu halten und so unerwünschte Nebenreaktionen auf ein Minimum zu beschränken.
   Bevorzugt wird die Spaltung in einer kombinierten Spalt- und Rektifizierkolonne durchgeführt, die für die Energiezufuhr im Sumpf mit einem Fallfilmverdampfer, im unteren Drittel mit einer Einrichtung zum zusätzlichen Energieeintrag bzw. zur Energierückgewinnung, im oberen Drittel mit einer Einrichtung zum Abzug von vorzugsweise Roh-Diisocyanat und am Kopf mit einem Kondensator für den Rückfluss und den Abzug von reinem Alkohol ausgestattet ist.
f) Die bei der thermischen Spaltung gebildeten Spaltprodukte, die sich vor allem aus Alkohol, Diisocyanat und partiell gespaltenen Diurethanen zusammensetzen, werden durch Rektifikation bei Temperaturen von 95 bis 260 °C, vorzugsweise 110 bis 245 °C und einem Druck von 0,5 bis 250 mbar, vorzugsweise 1 bis 100 mbar, in Alkohol und in eine rohe Diisocyanatmischung, bestehend aus cycloaliphatischem Diisocyanat, partiell gespaltenem cycloaliphatischem Diisocyanat und gegebenenfalls geringen Anteilen an cycloaliphatischen Diurethan, getrennt. Diese Trennung kann beispielsweise in der Spaltkolonne der obengenannten kombinierten Spalt- und Rektifizierkolonne durchgeführt werden.
g) Die vorzugsweise durch Rektifikation erhaltene rohe Mischung bestehend aus cycloaliphatischem Diisocyanat, partiell gespaltenem cycloaliphatischem Diurethan und gegebenenfalls geringen Anteilen an cycloaliphatischen Diurethan, wird durch Destillation bei einer Temperatur von 95 bis 260 °C, vorzugsweise 110 bis 245 °C und unter einem Druck von 0,5 bis 150 mbar, vorzugsweise 1 bis 75 mbar, gereinigt, wobei die anfallenden Fraktionen zurückgeführt oder als Reinprodukt isoliert werden.
h) Die Sumpfausschleusung aus der Deblockierungsstufe e) wird partiell oder vollständig mit dem Alkohol aus der Rektifikationsstufe f) zusammengeführt, wobei das Molverhältnis von NCO-Gruppen und OH-Gruppen bis zu 1 : 100, vorzugsweise 1 : 20 und besonders bevorzugt 1 : 10 beträgt, und die Reaktionsmischung in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt. Die Umsetzung kann in einer kontinuierlichen Kesselkaskade oder in einem Rohrreaktor durchgeführt werden. Als Katalysatoren kommen grundsätzlich alle Kontakte in Frage, die die NCO/OH-Reaktion fördern. Beispielhaft seien Zinnoctoat, Dibutylzinnlaurat, Zinndichlorid, Zinkdichlorid, Kupferchlorid, Kupferdichlorid, Eisendichlorid, Eisentrichlorid und Triethylamin aufgeführt.
i) Der Reurethanisatstrom aus Stufe h) wird in einen Wertstoff- und einen Abfallstrom aufgetrennt und der an Hochsiederkomponenten reiche Abfallstrom aus dem Prozess ausgeschleust und verworfen. Die Auftrennung der beiden Stoffströme erfolgt vorzugsweise destillativ mit Hilfe eines Dünnschicht- oder Kurzwegverdampfers, bei einer Temperatur von 180 bis 270 °C, vorzugsweise 200 bis 250 °C und unter einem Druck von 0,01 bis 10 mbar, vorzugsweise 0,02 bis 5 mbar. Der Wertstoffstrom, der die monomeren Diurethane und die leichter siedenden Nebenprodukte enthält, fällt als Destillat an. Der an hochsiedenden Komponenten reiche Abfallstrom fällt als Rückstand an und wird aus dem Herstellverfahren ausgeschleust und üblicherweise als stofflich nicht verwertbares Material verworfen. Alternativ, aber nicht bevorzugt, kann die Auftrennung in Wert- und Abfallstoff auch durch Extraktion erfolgen. Als Extraktionsmittel ist beispielsweise überkritisches Kohlendioxid geeignet.
   Optional kann der Reurethanisatstrom vor der oben beschriebenen destillativen Aufreinigung auch in zwei Teilströme aufgeteilt werden, von denen einer direkt der Reinigungsstufe c) zugeführt wird. Die Aufteilung der beiden Ströme kann im Verhältnis 99 : 1 bis 1 : 99, bevorzugt 99 : 5 bis 5 : 95 erfolgen. Optional kann der zur Hochsiederabtrennung führende Reurethanisatstrom zunächst partiell oder vollständig von überschüssigem Alkohol befreit werden. Dies erfolgt vorzugsweise destillativ. Der abgetrennte Alkohol kann wahlweise in Stufe a) und/oder Stufe c) zurückgeführt werden.
j) Ein Teil der Sumpffraktion der Reindestillation g) wird kontinuierlich ausgeschleust und optional in die Deblockierungsstufe e) oder in die Urethanisierungsstufe h) zurückgeführt. Die Rückführung in die Urethanisierungsstufe ist bevorzugt. Die Menge der Ausschleusung beträgt 0,1 bis 50 Gew.-%, vorzugsweise 0,2 bis 25 Gew.-% des Zulaufs an rohem Diisocyanat in die Reindestillationsstufe.
k) Die Kopffraktion der Reindestillationstufe g) kann vorworfen oder vorzugsweise in die Urethanisierungsstufe h) zurückgeführt werden. Die Menge der pro Zeiteinheit abgeführten Kopffraktion beträgt 0,1 bis 3 Gew.-%, vorzugsweise 0,3 bis 1 Gew.-% des Zulaufs an rohem Diisocyanat in die Reindestillation.
l) Der aufgereinigte Reurethanisatstrom aus Stufe i) wird in die Leicht- und Mittelsiederabtrennung c) und/oder die Diurethanherstellung b) oder die Diurethanspaltung e) zurückgeführt.

Mit dem erfindungsgemäßen mehrstufigen Verfahren zur kontinuierlichen Herstellung von cycloaliphatischen Diisocyanaten unter Rückführung und Ausschleusung der Nebenprodukte kann für destillierbare cycloaliphatische Diisocyanate eine mit hoher Selektivität störungsfrei ablaufende Reaktion gewährleistet werden. Das erfindungsgemäße Verfahren eignet sich insbesondere zur Herstellung von cycloaliphatischen Diisocyanaten mit 4 bis 18, vorzugsweise 5 bis 15 Kohlenstoffatomen wie 1,4-Diisocyanatocyclohexan, 4,4'-Methylendicyclohexyldiisocyanat (4,4'-H₁₂MDI), 2,2'-Methylendicyclohexyldiisocyanat (2,2'-H₁₂MDI), 2,4'-Methylendi-cyclohexyldiisocyanat (2,4'-H₁₂MDI) oder auch Mischungen der vorgenannten isomeren Methylendicyclohexyldiisocyanate (H₁₂MDI), wie sie zum Beispiel naturgemäß bei der Umwandlung von perhydriertem MDA in H₁₂MDI anfallen. Ganz besonders bevorzugt werden 4,4'-Methylendicyclohexyldiisocyanat sowie beliebige Mischungen aus 4,4'-H₁₂MDI, 2,4-H₁₂MDI und 2,2'-H₁₂MDI hergestellt.

Die hergestellten cycloaliphatischen Diisocyanate eigenen sich bestens zur Herstellung von Urethan-, Isocyanurat-, Amid- und/oder Harnstoffgruppen enthaltenden Kunststoffen nach dem Polyisocyanat-Polyadditionsverfahren. Sie finden darüber hinaus Verwendung zur Herstellung von mit Urethan-, Biuret-, und/oder Isocyanuratgruppen modifizierten Polyisocyanatmischungen. Derartige Polyisocyanatmischungen aus cycloaliphatischen Diisocyanaten werden insbesondere zur Herstellung von hochwertigen, lichtbeständigen Polyurethanbeschichtungen eingesetzt.

Die Erfindung wird durch folgendes Beispiel näher erläutert.

### Beispiel

### Beispiel 1: Erfindungsgemäße Herstellung von Methylendicyclohexyldiisocyanat (H₁₂MDI) aus perhydriertem Methylendiphenyldiamin und Harnstoff in Gegenwart von n-Butanol.

Der oberste Boden eines Destillationsreaktors wurden stündlich mit 281,2 g H₁₂MDA, 164,9 g Harnstoff und 595 g n-Butanol beaufschlagt und die Reaktionsmischung unter kontinuierlicher Entfernung des freigesetzten Ammoniaks bei Normalkdruck, 135 °C und einer mittleren Verweilzeit von 8 Stunden gekocht. Die im Sumpf des Destillationsreaktors anfallende Lösung von Bisharnstoff in Butanol wurde über einen Wärmetauscher auf 190 °C vorgeheizt, auf den obersten Boden eines Druckdestillationsreaktors geführt und bei 11 bis 14 bar, 220 °C und mit einer mittleren Verweilzeit von 10,5 h weiter umgesetzt. Im Sumpf des Druckdestillationsreaktors wurden stündlich 540,1 g n-Butanol eingespeist und die zusammen mit dem freigesetzten Ammoniak am Kopf abgezogene Alkoholmenge so gewählt, dass sie dem Alkoholeintrag im Sumpf entsprach. Der Reaktoraustrag wurde, zusammen mit dem Stoffstrom aus der Hochsiederabtrennung, im Flash-Behälter bei 55 mbar und anschließender Dünnschichtverdampfung bei 220 °C und 2 mbar von überschüssigem Alkohol, Leicht- und Mittelsiedern befreit und die verbleibenden 771,1 g/h Bis-(4-butoxycarbonylamino-cyclohexyl)-methan (H₁₂MDU) als Schmelze (140 °C) in die Wälzung des Fallfilmverdampfers der Spalt- und Rektifizierkolonne gefahren, wobei die Deblockierungsreaktion bei einer Temperatur von 239 °C und einem Sumpfdruck von 10 mbar in Gegenwart einer stationären Konzentration an Zinndichlorid von 16 ppm durchgeführt wurde. Die Spaltgase H₁₂MDI und Butanol wurden in zwei hintereinandergeschalteten Kondensatoren bei 85 °C und -25 °C auskondensiert. Das gewonnene, etwa 97 %ige Roh- H₁₂MDI wurde einer Reindestillation zugeführt, wobei 320,9 g/h H₁₂MDI mit einer Reinheit von > 99,5 % erhalten wurden, was einer auf das Amin bezogenen Ausbeute von 92 % entspricht. 228,9 g/h Butanol fielen als Kopfprodukt der Spalt- und Rektifizierkolonne an. Zur Aufrechterhaltung der Massenkonstanz innerhalb der Spalt- und Rektifizierkolonne und Vermeidung von Belegungen und Verstopfungen der Spaltapparatur wurde kontinuierlich ein Teilstrom aus dem Wälzkreislauf ausgeschleust und zusammen mit 23,7 g/h Sumpfauskreisung aus der H₁₂MDI-Reindestillation sowie dem Kopfprodukt aus der Spalt- und Rektifizierkolonne vereinigt und reurethanisiert. Der Reurethanisatstrom wurde durch Flash-Verdampfung bei 40 mbar von überschüssigem Butanol befreit und mittels eines Kurzwegverdampfers bei 230°C und einem Druck von 0,04 mbar in einen Hochsieder reichen Abfallstrom und einen Wertstoffstrom aufgetrennt. Die 229,9 g/h Wertstoffstrom wurden zusammen mit dem Reaktoraustrag der Diurethanherstellung dem Flash-Behälter zugeführt.

## Patentansprüche

1. Mehrstufiges Verfahren zur kontinuierlichen Herstellung von cycloaliphatischen Diisocyanaten, durch Umsetzung von cycloalipahtischen Diaminen mit Kohlensäurederivaten und Alkoholen zu cycloaliphatischen Diurethanen und anschließender thermischen Spaltung der Diurethane zu cycloaliphatischen Diisocyanaten,
**dadurch gekennzeichnet,**
**dass** die Bildung der Diurethane zweistufig ausgeführt ist, das von Leicht-, Mittelsiedern befreite Diurethan unter Freisetzung des gewünschten Diioscyanats thermisch gespalten wird, ein Teil des Spaltsumpfes der Spaltapparatur kontiniuerlich ausgeschleust und mit Alkohol reurethanisiert wird, hieraus die Hochsieder abgetrennt werden und das so gereinigte Reurethanisat in den Prozess recycliert wird.

2. Mehrstufiges Verfahren zur kontinuierlichen Herstellung von cycloaliphatischen Diisocyanaten der Formel (I)
OCN-R-NCO
wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden Isocyanatgruppen direkt an einem Kohlenwasserstoffcyclus gebunden und zwischen ihnen mindestens 3 Kohlenstoffatome angeordnet sind, durch Umsetzung cycloaliphatischer Diamine mit Kohlensäurederivaten und Alkoholen zu Diurethanen, und deren thermische Spaltung, wobei
a) cycloaliphatische Diamine der Formel (II)
H₂N-R-NH₂
wobei R für einen zweiwertigen cycloaliphatischen Kohlenwasserstoffrest mit 4 bis 18, vorzugsweise 5 bis 15 Kohlenstoffatomen steht, wobei die beiden Stickstoffatome direkt an mindestens einem Kohlenwasserstoffcyclus gebunden und zwischen ihnen mindestens 3 Kohlenstoffatome angeordnet sind, mit Harnstoff und in Gegenwart von Alkoholen der Formel (III)
R¹-OH
wobei R¹ für einen Rest steht, wie er nach Entfernung der Hydroxylgruppe aus einem primären oder sekundären (cyclo)aliphatischen Alkohol mit 3 bis 8 Kohlenstoffatomen verbleibt,
in Abwesenheit oder Gegenwart von Katalysatoren zu Cycloalkylenbisharnstoffen der Formel (IV)
H₂N-OC-HN-R-NH-CO-NH₂
wobei R für einen zweiwertigen cycloaliphatischen Kohlenwasserstoffrest mit 4 bis 18, vorzugsweise 5 bis 15 Kohlenstoffatomen steht, mit der Maßgabe, dass die beiden R flankierenden Stickstoffatome direkt an einem Kohlenwasserstoffcyclus gebunden und zwischen ihnen mindestens 3 Kohlenstoffatome angeordnet sind, umgesetzt werden und das entstehende Ammoniak gleichzeitig kontinuierlich abgetrennt wird;
b) der anfallende Roh-Cycloalkylenbisharnstoff in einem zweiten Reaktor mit dem in a) als Lösemittel eingesetzten Alkohol der Formel (III) unter kontinuierlicher Austreibung des freigesetzten Ammoniaks in Cycloalkylendiurethan der Formel (V)
R¹O-OC-HN-R-NH-CO-OR¹
überführt wird;
c) aus der erhaltenen Reaktionsmischung der Alkohol, die Dialkylcarbonate und/oder Carbamidsäurealkylester abgetrennt und der Alkohol in die Reaktionsstufe a) zurückführt wird;
d) auf eine Abtrennung der in der erhaltenen Reaktionsmischung gegebenenfalls enthaltenen hochsiedenden Rückstände vollständig oder partiell verzichtet wird;
e) die über die Schritte c) und d) aufgereinigte Diurethane enthaltende Reaktionsmischung in Gegenwart eines Katalysators kontinuierlich und lösemittelfrei bei Temperaturen von 180 bis 280 °C, vorzugsweise 200 bis 260 °C, und unter einem Druck von 0,1 bis 200 mbar, vorzugsweise 0,2 bis 100 mbar thermisch so gespalten wird, dass ein Teil des Reaktionsgemisches von 10 bis 60 Gew.-% bezogen auf den Feed, vorzugsweise 15 bis 45 Gew.-% bezogen auf den Feed, ständig aus dem Sumpf ausgeschleust wird;
f) die Spaltprodukte durch Rektifikation in ein rohes Diisocyanat und Alkohol getrennt werden;
g) das rohe cycloaliphatische Diisocyanat durch Destillation gereinigt und die Reinproduktfraktion isoliert wird;
h) die Sumpfausschleusung aus e) partiell oder vollständig mit dem Alkohol aus f) in Gegenwart oder Abwesenheit von Katalysatoren innerhalb von 1 bis 150 min, vorzugsweise 3 bis 60 min, bei Temperaturen von 20 bis 200 °C, vorzugsweise 50 bis 170 °C und bei einem Druck von 0,5 bis 20 bar, vorzugsweise 1 bis 15 bar, umgesetzt (reurethanisiert) wird und das Molverhältnis von NCO-Gruppen und OH-Gruppen bis zu 1 : 100, vorzugsweise 1 : 20 und besonders bevorzugt 1 : 10 beträgt;
i) der Reurethanisatstrom aus h) in einen Wertstoff- und einen Abfallstrom aufgetrennt und der an Hochsiederkomponenten reiche Abfallstrom aus dem Prozess ausgeschleust und verworfen wird;
j) ein Teil der Sumpffraktion der Reindestillation g) kontinuierlich ausgeschleust und in die Spaltreaktion e) und/oder in die Urethanisierungsstufe h) geführt wird;
k) optional die bei der Reindestillation des rohen cycloaliphatischen Diisocyanats anfallende Kopffraktion ebenfalls in die Urethanisierungsstufe h) zurückgeführt wird;
l) der aufgereinigte Reurethanisatstrom aus i) in Stufe b) und/oder c) oder e) zurückgeführt wird.

3. Mehrstufiges Verfahren nach Anspruch 1 oder 2,
**dadurch gekennzeichnet**,
das als cycloaliphatisches Diamin 4,4`-Methylendicyclohexyldiamin, 2,4-Methylendicyclohexyldiamin und 2,2'-Methylendicyclohexyldiamin sowie auch beliebige Mischungen mindestens zweier dieser Isomere eingesetzt werden.

4. Mehrstufiges Verfahren nach Anspruch 1 oder 2,
**dadurch gekennzeichnet,**
**dass** als cycloaliphatisches Diamin 4,4'-Methylendicyclohexyldiamin und/oder isomere cycloaliphatische Diamine eingesetzt werden.

5. Mehrstufiges Verfahren nach Anspruch 1,
**dadurch gekennzeichnet,**
**dass** als cycloaliphatisches Diamin 1,4-Diaminocyclohexan eingesetzt wird.

6. Verfahren nach mindestens einem der vorherigen Ansprüche,
**dadurch gekennzeichnet,**
**dass** die Stufe a) in einem Reaktor bei 100 bis 145 °C und einem Druck von 0,7 bis 1,8 bar durchgeführt wird.

7. Verfahren nach mindestens einem der vorherigen Ansprüche,
**dadurch gekennzeichnet,**
**dass** die Stufe a) in einem Destillationsreaktor durchgeführt wird.

8. Verfahren nach mindestens einem der vorherigen Ansprüche,
**dadurch gekennzeichnet,**
**dass** die Umsetzung in Stufe a) in einem Molverhältnis von Diamin : Harnstoff : Alkohol von 1 : 2,0 bis 2,4 : 3 bis 10 erfolgt.

9. Verfahren nach mindestens einem der vorherigen Ansprüche,
wobei in Stufe a) die Edukte kontinuierlich auf den obersten Boden aufgegeben werden und das freigesetzte Ammoniak durch Alkoholbrüden, die im Sumpf des Destillationsreaktor eingeführt werden, ausgetrieben wird.

10. Verfahren nach mindestens einem der vorherigen Ansprüche,
wobei die Verweilzeit der Edukte in Stufe a) 4 bis 10, vorzugsweise 5 bis 9 Stunden beträgt.

11. Verfahren nach mindestens einem der vorherigen Ansprüche,
**dadurch gekennzeichnet,**
**dass** die Stufe b) in einem Druckdestillationsreaktor durchgeführt wird.

12. Verfahren nach mindestens einem der vorherigen Ansprüche,
**dadurch gekennzeichnet,**
**dass** in Stufe b) bei einem molaren Verhältnis von Bisharnstoff zu Alkohol von 1 : 5 bis 12 verfahren wird.

13. Verfahren nach mindestens einem der vorherigen Ansprüche,
**dadurch gekennzeichnet,**
**dass** der Stoffstrom aus a) vorzugsweise kontinuierlich auf den obersten Boden des Reaktors der Stufe b) gefahren wird.

14. Verfahren nach mindestens einem der vorherigen Ansprüche,
**dadurch gekennzeichnet,**
**dass** in Stufe b) bei Reaktionstemperaturen von 140 bis 270 °C, vorzugsweise 160 bis 250 °C und unter einem Druck, der 5 bis 20 bar, vorzugsweise 7 bis 15 bar beträgt, umgesetzt wird.

15. Verfahren nach mindestens einem der vorherigen Ansprüche,
**dadurch gekennzeichnet,**
**dass** die Umsetzung in Stufe b) innerhalb von 2 bis 20 Stunden, vorzugsweise 8 bis 15 Stunden, stattfindet.

16. Verfahren nach mindestens einem der vorherigen Ansprüche,
**dadurch gekennzeichnet,**
**dass** die Umsetzung in Stufe a) und/oder in Stufe b) in Gegenwart von Katalysatoren durchgeführt wird.

17. Verfahren nach mindestens einem der vorherigen Ansprüche,
**dadurch gekennzeichnet,**
**dass** in Stufen a) und b) Alkohole mit 1 bis 6 Kohlenstoffatomen eingesetzt werden.

18. Verfahren nach mindestens einem der vorherigen Ansprüche,
**dadurch gekennzeichnet,**
**dass** in Stufen a) und b) Butanol verwendet wird.

19. Verfahren nach mindestens einem der vorherigen Ansprüche,
**dadurch gekennzeichnet,**
**dass** die Stufe c) zweistufig durchgeführt wird.

20. Verfahren nach Anspruch 19,
**dadurch gekennzeichnet,**
**dass** auf der ersten Stufe die Reaktionsmischung vom Druckniveau der Reaktionsstufe b) auf einen Druck von 1 bis 500 mbar, vorzugsweise 2 bis 150 mbar, entspannt wird.

21. Verfahren nach Anspruch 19 oder 20,
**dadurch gekennzeichnet,**
**dass** im zweiten Schritt der flüssige Austrag durch Dünnschichtverdampfung bei 180 °C bis 250 °C, vorzugsweise 200 ° bis 230 °C, und einem Druck von 0,1 mbar bis 20 mbar, vorzugsweise 1 mbar bis 10 mbar, von gegebenenfalls vorhandenem restlichen Alkohol sowie Mittelsiedern wie Dialkylcarbonaten und/oder Caramidsäurealkylestern befreit wird.

22. Verfahren nach den Ansprüchen 19, 20 oder 21,
**dadurch gekennzeichnet,**
**dass** die Brüden der Stufe c) nach weiterer destillativer Reinigung in die Reaktionsstufe a) zugeführt werden.

23. Verfahren nach mindestens einem der vorhergehenden Ansprüche,
**dadurch gekennzeichnet,**
**dass** die Trennung in Stufe d), falls angewendet, bei einer Temperatur von 180 bis 260 °C, vorzugsweise 200 bis 240 °C und unter einem Druck von 0,01 bis 10 mbar, vorzugsweise 0,02 bis 5 mbar durchgeführt wird.

24. Verfahren nach mindestens einem der vorhergehenden Ansprüche,
**dadurch gekennzeichnet,**
**dass** die Stufe d), falls angewendet, mit Hilfe eines Dünnschicht- oder Kurzwegverdampfers durchgeführt wird.

25. Verfahren nach mindestens einem der vorhergehenden Ansprüche,
**dadurch gekennzeichnet,**
**dass** die Nebenprodukte aus Stufe d), falls angewendet, ausgeschleust und verworfen werden.

26. Verfahren nach mindestens einem der vorhergehenden Ansprüche,
**dadurch gekennzeichnet,**
**dass** der Stoffstrom aus Stufe c) vor seiner Überfixhrung in Stufe d), falls diese angewendet wird, so verarbeitet wird, dass er vor seiner destillativen Aufreinigung in zwei Teilströmen aufgeteilt wird, von denen ein Teilstrom direkt der Deblockierungsreaktion (siehe e) zugeführt wird.

27. Verfahren nach mindestens einem der vorhergehenden Ansprüche,
**dadurch gekennzeichnet,**
**dass** die thermisch induzierte Diurethanspaltung der Stufe e) in Röhrenöfen oder vorzugsweise Verdampfern, beispielsweise Fallfilm-, Dünnschicht- oder Bulkverdampfern, wie z. B. Robertverdampfern, Herbertverdampfern, caddle-typ-Verdampfern, Oskarverdampfern und Heizkerzenverdampfern durchgeführt wird.

28. Verfahren nach mindestens einem der vorhergehenden Ansprüche,
**dadurch gekennzeichnet,**
**dass** die Stufe e) in einer kombinierten Spalt- und Rektifizierkolonne durchgeführt wird.

29. Verfahren nach mindestens einem der vorhergehenden Ansprüche,
**dadurch gekennzeichnet,**
**dass** in Stufe e) in Gegenwart von Katalysatoren bei Temperaturen von 180 bis 280 °C, vorzugsweise 200 bis 260 °C, und unter einem Druck von 0,1 bis 200 mbar, vorzugsweise 0,2 bis 100 mbar kontinuierlich thermisch gespalten wird.

30. Verfahren nach mindestens einem der vorhergehenden Ansprüche,
**dadurch gekennzeichnet,**
**dass** in Stufe e) lösemittelfrei in flüssiger Phase gespalten wird.

31. Verfahren nach mindestens einem der vorhergehenden Ansprüche,
**dadurch gekennzeichnet,**
**dass** die Stufe e) in Gegenwart von Katalysatoren durchgeführt wird.

32. Verfahren nach mindestens einem der vorhergehenden Ansprüche,
**dadurch gekennzeichnet,**
**dass** in Stufe e) der Umsatz von Diurethan zu Diisocyanat in Abhängigkeit vom verwendeten Diurethan frei gewählt wird, bevorzugt in einem Bereich von 10 bis 95 Gew.-%, vorzugsweise 35 bis 85 Gew.-% der zugeführten Diurethanmenge (Feed) liegt.

33. Verfahren nach mindestens einem der vorhergehenden Ansprüche,
**dadurch gekennzeichnet,**
**dass** in Stufe e) ein Teil der Reaktionsmischung, der nicht umgesetzten Diurethane, hochsiedende Nebenprodukte und andere wieder verwertbare und unverwertbare Nebenprodukte enthält, kontinuierlich ausgeschleust wird.

34. Verfahren nach Anspruch 33,
**dadurch gekennzeichnet,**
**dass** die Menge der Ausschleusung 10 bis 60 Gew.-%, vorzugsweise 15 bis 45 Gew.-%, bezogen auf den Feed, beträgt.

35. Verfahren nach mindestens einem der vorhergehenden Ansprüche,
**dadurch gekennzeichnet,**
**dass** Stufe f) in einer kombinierten Spalt- und Rektifizierkolonne durchgeführt wird.

36. Verfahren nach mindestens einem der vorhergehenden Ansprüche,
**dadurch gekennzeichnet,**
**dass** bei Temperaturen von 95 °C bis 260 °C, vorzugsweise 110 °C bis 245 °C und einem Druck von 0,5 mbar bis 250 mbar, vorzugsweise 1 mbar bis 200 mbar verfahren wird.

37. Verfahren nach mindestens einem der vorhergehenden Ansprüche,
**dadurch gekennzeichnet,**
**dass** in Stufe g) die aus Stufe f) erhaltene rohe Fraktion bestehend aus cycloaliphatischem Diisocyanat, partiell gespaltenem cycloaliphatischem Diurethan und gegebenenfalls geringen Anteilen an cycloaliphatischen Diurethan, wird durch Destillation bei einer Temperatur von 95 bis 260 °C, vorzugsweise 110 bis 245 °C und unter einem Druck von 0,5 mbar bis 150 mbar, vorzugsweise 1 mbar bis 75 mbar gereinigt wird.

38. Verfahren nach mindestens einem der vorhergehenden Ansprüche,
**dadurch gekennzeichnet,**
**dass** die in Stufe g) anfallende Fraktion als Reinprodukt isoliert oder in Stufe h) zurückgeführt wird.

39. Verfahren nach mindestens einem der vorhergehenden Ansprüche,
**dadurch gekennzeichnet,**
**dass** in Stufe i) bei einer Temperatur von 180 °C bis 270 °C, vorzugsweise 200 °C bis 250 °C und unter einem Druck von 0,01 mbar bis 10 mbar, vorzugsweise 0,02 mbar bis 5 mbar verfahren wird.

40. Verfahren nach mindestens einem der vorhergehenden Ansprüche,
**dadurch gekennzeichnet,**
**dass** die Stufe i) durch Extraktion erfolgt.

41. Verfahren nach mindestens einem der vorhergehenden Ansprüche,
**dadurch gekennzeichnet,**
**dass** in Stufe i) der Reurethanisatstrom vor der destillativen Aufreinigung in zwei Teilströme aufgeteilt wird, von denen einer direkt der Reinigungsstufe c) zugeführt wird.

42. Verfahren nach mindestens einem der vorhergehenden Ansprüche,
**dadurch gekennzeichnet,**
**dass** die Aufteilung der beiden Teilströme im Verhältnis 99 : 1 bis 1 : 99, bevorzugt 95 : 5 bis 5 : 95 erfolgt.

43. Verfahren nach mindestens einem der vorhergehenden Ansprüche,
**dadurch gekennzeichnet,**
**dass** die Stufe i) in einer kontinuierlichen Kesselkaskade oder in einem Rohrreaktor durchgeführt wird.

44. Verfahren nach mindestens einem der vorhergehenden Ansprüche,
**dadurch gekennzeichnet,**
**dass** die Umsetzung in Stufe h) in Gegenwart von Katalysatoren aus der Gruppe der Sn- und/oder Zn- und/oder Cu-Carboxylate oder -Halogenide, der tert. Amine, und/oder der Eisenhalogenide erfolgt.

45. Verfahren nach mindestens einem der vorhergehenden Ansprüche,
**dadurch gekennzeichnet,**
**dass** in Stufe j) die Rückführung in die Deblockierungsstufe e) oder in die Urethanisierungsstufe h) erfolgt.

46. Verfahren nach mindestens einem der vorhergehenden Ansprüche,
**dadurch gekennzeichnet,**
**dass** in Stufe j) die Menge der Ausschleusung 0,1 bis 50 Gew.-%, vorzugsweise 0,2 bis 25 Gew.-% des Zulaufs an rohem Polyisocyanat in die Reindestillationsstufe beträgt.

47. Verfahren nach mindestens einem der vorhergehenden Ansprüche,
**dadurch gekennzeichnet,**
**dass** der zur Hochsiederabtrennung i) führende Reurethanisatstrom aus h) zunächst partiell oder vollständig von überschüssigem Alkohol befreit und der abgetrennte Alkohol wahlweise in Stufe a) und/oder c) zurückgeführt wird.

48. Verfahren nach mindestens einem der vorhergehenden Ansprüche,
**dadurch gekennzeichnet,**
**dass** der aufgereinigte Reurethanisatstrom aus Stufe i) in Stufe b) und/oder c) oder e) zurückgeführt wird.

49. Verfahren nach mindestens einem der vorhergehenden Ansprüche,
**dadurch gekennzeichnet,**
**dass** 1,4-Diisocyanatocyclohexan, 4,4'-Methylendicyclohexyldiisocyanat, 2,2'-Methylendicyclohexyldiisocyanat, 2,4'-Methylendicyclohexyldiisocyanat oder auch beliebige Mischungen mindestens zweier isomerer Methylendicyclohexyldiisocyanate hergestellt werden.

50. Verfahren nach mindestens einem der vorhergehenden Ansprüche,
**dadurch gekennzeichnet,**
**dass** Diamine ausgewählt aus 1,3- und 1,4-Diaminomethylcyclohexan, Hexandiamin-1,6, 2,2,4- bzw. 2,4,4-Trimethylhexanamin-1,6 und 3-Aminomethyl-3,5,5-trimethylcyclohexylamin eingesetzt werden.
